(19) Europäisches Patentamt
European Patent Office
Office européen des brevets

(11) Publication number: **0 148 517 B1**

(12) **EUROPEAN PATENT SPECIFICATION**

(45) Date of publication of patent specification: **21.09.88**

(51) Int. Cl.⁴: **E 21 B 43/22,** C 11 D 1/37

(21) Application number: **84201676.8**

(22) Date of filing: **20.11.84**

(54) **Use of olefin sulphonate compositions in enhanced oil recovery processes.**

(30) Priority: **28.11.83 GB 8331733**

(43) Date of publication of application:
**17.07.85 Bulletin 85/29**

(45) Publication of the grant of the patent:
**21.09.88 Bulletin 88/38**

(84) Designated Contracting States:
**DE FR GB IT NL**

(56) References cited:
**DE-A-1 617 064**
**DE-A-2 523 589**
**GB-A-2 095 309**
**US-A-4 086 964**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Craven, Sheila Mairi**
**Chopinstraat 4**
**NL-1921 XK Akersloot (NL)**
Inventor: **Fernley, George William**
**Badhuisweg 3**
**NL-1031 CM Amsterdam (NL)**

(74) Representative: **Aalbers, Onno et al**
**P.O. Box 302**
**NL-2501 CH The Hague (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 148 517 B1

**Description**

This invention relates to the use of olefin sulphonate compositions in enhanced oil recovery processes, particularly from drive processes of enhanced oil recovery.

Foam drive processes for enhanced oil recovery are described in US Patent 4,086,964, 4,161,217 and 4,393,937. Such processes typically achieve displacement of oil within an oil-containing subterranean reservoir by flowing a steam-containing fluid containing a surfactant component through a relatively steam-permeable zone within the reservoir. In the process of US Patent 4,393,937, the surfactant component comprises of olefin sulphonate. The olefin sulphonate is conveniently used as a 30 %w solution in water. Alpha-olefin sulphonates, e.g. $C_{10-24}$, preferably $C_{14-20}$, and advantageously $C_{16-18}$, are disclosed as being particularly suitable. However, although 30 %w aqueous solutions of sodium $C_{16-18}$ alpha-olefin sulphonates have low viscosites (typically ca 8.5 $mm^2/s$ at 20°C), they are not stable on storage for 48 hours at 20°C. Indeed the minimum storage temperature (MST) is typically 30°C, which necessitates transport and storage at elevated temperatures, generally in insulated containers. US Patent 4,161,217 specifically discloses alkyl benzene sulphonates for such processes. However, in addition to having relatively high MST (typically greater than 30°C), 30% aqueous solutions of alkyl benzene sulphonates are typically highly viscous (e.g. greater than 6000 $mm^2/s$ at 20°C).

W. German Offenlegungsschrift 2,523,589 discloses a dishwashing composition which comprises (a) $C_{8-20}$ alpha-olefin sulphonate and (b) $C_{8-24}$ secondary alkanol sulphonates and/or ($C_{9-18}$ alkyl) aryl sulphonates, wherein the weight ratio a:b is in the range 50:50 to 10:90.

W. German Offenlegungsschrift 1,617,064 discloses certain washing compositions comprising an olefin sulphonate, a potassium xylene sulphonate, tetra potassium pyrophosphate and other ingredients, but, however, is silent about the use in an enhanced oil recovery process.

Canadian Patent 1,031,686 discloses a water-flooding component for enhanced oil recovery, which comprises an aqueous composition containing a maximum of 5 %w of each of (a) an oil-soluble petroleum sulphonate, (b) a $C_{8-30}$, preferably $C_{12-18}$, alpha-olefin sulphonate and (c) an oxyalkylated alcohol.

It has now surprisingly been found possible to provide an olefin sulphonate composition having a high concentration of surfactant, which has a minimum storage temperature not greater than 20°C while having a viscosity at 20°C of less than 1000 $mm^2/s$. Such compositions are of sufficiently low viscosity to be readily transported and handled (e.g. transferred from one container to another), and do not have to be stored at disadvantageous elevated temperatures.

The present invention therefore relates to the use of an olefin sulphonate composition in an enhanced oil recovery process characterized in that the composition comprises an aqueous solution containing 15 to 35 %w of a blend of (a) $C_{12-22}$ linear alpha-olefin sulphonate and (b) alkyl aryl sulphonate of general formula

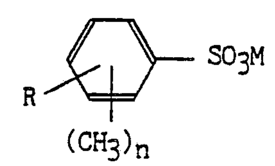

(I)

where n is 0, 1 or 2, R is a $C_{8-18}$ alkyl group and M is an alkali metal or ammonium ion, the weight ratio (a):(b) being in the range 90:10 to 60:40.

Linear alpha-olefin sulphonates are known compounds and various examples thereof are described in US Patent 4,393,937, together with outline process for their preparation. Preferably the olefin sulphonate is a $C_{14-20}$ linear alpha-olefin sulphonate, more preferably a $C_{16-18}$ linear alpha-olefin suphonate. Suitable olefin sulphonates are conveniently prepared by sulphonation, hydrolysis and neutralisation of $C_{12-22}$ linear alpha-olefins prepared by the "SHELL" Higher Olefins Process ("SHELL" is a registered trade mark).

Compounds of formula I are alkyl benzene sulphonates (n is 0), alkyl toluene sulphonate (n is 1) and alkyl xylene sulphonates. Such compounds are also known. Preferably n is 0. It is further preferred for R to be a $C_{10-14}$ alkyl group. Conveniently the compounds of formula I are those prepared by alkylation of benzene, toluene or xylene with linear alpha-olefins prepared by the "SHELL" Higher Olefin Process ("SHELL" is a registered trade mark), followed by sulphonation and neutralisation.

The olefin sulphonate (a) and the alkyl aryl sulphonate (b) are alkali metal or ammonium salts. Alkali metal salts may be e.g. potassium or, preferably, sodium salts. Ammonium salts include those derived from ammonia and from primary, secondary or tertiary amines, for example amines bearing one, two or three $C_{1-6}$ alkyl or hydroxyalkyl moieties, e.g. triethanolamine. Alternative ammonium salts are quaternary salts such as tetramethyl ammonium salts. Sodium salts are very suitable.

Preferably the aqueous solution contains at least 20 %w, advantageously at least 25 %w, of the blend of (a) and (b). More preferably the aqueous solution contains substantially 30 %w of the blend of (a) and (b).

The invention will be further understood from the following illustrative Examples, in which parts and percentages are by weight (w/w) unless otherwise indicated.

Examples 1 to 4

A $C_{16-18}$ linear alpha-olefin sulphonate composition containing 30% active matter and sold by member companies of the Royal Dutch/Shell group of companies under the registered trade mark "ENORDET AOS/340", is prepared by sulphonation using vapourised sulphur trioxide, followed by hydrolysis and neutralisation to give the sodium salt, of a mixture of $C_{16-18}$ linear alpha-olefins prepared by the "SHELL" Higher Olefins Process ("SHELL" is a registered trade mark), and characterized by consisting of about 55% $C_{16}$, about 42% $C_{18}$ and not greater than 3% of each of $C_{14}$ and $C_{20}$ olefins, of which about 94% is linear terminal, about 3% branched terminal and about 2% linear terminal.

A ($C_{10-14}$ linear alkyl) benzene sulphonate solution containing 30% active matter was prepared by adding 100 g "DOBANIC ACID JN" (registered trade mark) (containing 97.5% active matter) to 72 g of 16.7% aqueous sodium hydroxide solution and diluting the resulting paste with water to give aqueous solution of 30% active matter. "DOBANIC ACID JN" is prepared by sulphonation of "DOBANE JN" (registered trade mark) detergent alkylate wherein typically 12% of the alkyl substituents are $C_{10}$, 28% are $C_{11}$, 27% are $C_{12}$, 25% are $C_{13}$ and 8% are $C_{14}$.

The resulting olefin sulphonate composition are alkyl benzene sulphonate solution were heated to 30°C to 60°C until homogeneous, and blends were prepared. In each case, of course, the active matter content was 30%.

The viscosities of the various compositions were measured using a Ubbelohde viscometer at 20°C according to ASTM D445.

Minimum storage temperature determination was as follows. The compositions were stored for at least 48 hours in a cooler. The temperature of the cooler was lowered by 1°C every 48 hours, and the temperature immediately prior to that at which precipitation was observed was noted as the minimum storage temperature (MST).

Constitution of the various compositions and results of viscosity and MST determinations are given in Table I following.

TABLE I

| Example | Weight ratio olefin sulphonate: alkyl benzene sulphonate | Viscosity ($mm^2/s$) | MST (°C) |
|---|---|---|---|
| Comparative | 100:0 | 8.5 | 30 |
| 1 | 90:10 | 11.5 | 20 |
| 2 | 80:20 | 28 | 15 |
| 3 | 70:30 | 77 | 11 |
| 4 | 60:40 | 256 | 8 |
| Comparative | 0:100 | 6840 | 34 |

It can readily be seen that the presence of the alkyl benzene sulphonate confers surprising benefit in terms of reduced MST, whilst viscosity remains conveniently low.

**Claims**

1. Use of olefin sulphonate composition in an enhanced oil recovery process characterized in that the composition comprises an aqueous solution containing 15 to 35 %w of a blend of (a) $C_{12-22}$ linear alpha-olefin sulphonate and (b) alkyl aryl sulphonate of general formula

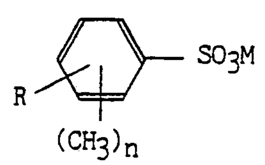

(I)

where n is 0, 1 or 2, R is a $C_{8-18}$ alkyl group and M is an alkali metal or ammonium ion, the weight ratio (a):(b) being in the range 90:10 to 60:40.

2. Use of a composition according to claim 1 characterized in that the olefin sulphonate is a $C_{14-20}$ linear alpha-olefin sulphonate.

3. Use of a composition according to claim 1 or 2 chracterized in that the olefin sulphonate is a $C_{16-18}$ linear alpha-olefin sulphonate.

4. Use of a composition according to claim 1, 2 or 3 characterized in that in formula I n is 0.

5. Use of a composition according to any of claims 1 to 4 characterized in that R is a $C_{10-14}$ alkyl group.

6. Use of a composition according to any of claims 1 to 5 characterized in that the olefin sulphonate and the alkyl aryl sulphonate are sodium salts.

7. Use of a composition according to any of claims 1 to 6 characterized in that aqueous solution contains at least 25 %w of the blend of (a) and (b).

8. Use of a composition according to any of claims 1 to 7 characterized in that the aqueous solution contains substantially 30 %w of the blend of (a) and (b).

**Patentansprüche**

1. Verwendung einer Olefinsulfonatzusammensetzung in einem Verfahren zur Steigerung der Ölgewinnung, dadurch gekennzeichnet, daß die Zusammensetzung eine wäßrige Lösung umfaßt, die 15 bis 35 Gew.-% einer Mischung enthält aus (a) linearem $C_{12-22}$ alpha-Olefin-Sulfonat und (b) Alkylarylsulfonat der allgeneinen Formel

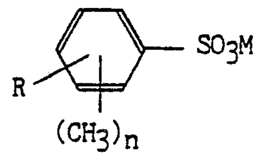

(I)

worin n 0,1 oder 2 ist, R eine $C_{8-18}$ Alkylgruppe ist und M eine Alkalimetall oder Ammoniumion ist, wobei das Gewichtsverhältnis (a):(b) im Bereich von 90:10 bis 60:40 liegt.

2. Verwendung einer Zusammensetzung nach Anspruch 1, dadurch gekennzeichnet, daß das Olefinsulfonat ein lineares $C_{14-20}$ alpha-Olefin-Sulfonat ist.

3. Verwendung einer Zusammensetzung nach Anspruch 1 oder 2, dadurch gekennzeichnet, daß das Olefinsulfonat ein lineares $C_{16-18}$ alpha-Olefin-Sulfonat ist.

4. Verwendung einer Zusammensetzung nach Anspruch 1, 2 oder 3, dadurch genennzeichnet, daß in Formel I n 0 ist.

5. Verwendung einer Zusammensetzung nach einem der Ansprüche 1 bis 4, dadurch gekennzeichnet, daß R eine $C_{10-14}$ Alkylgruppe ist.

6. Verwendung einer Zusammensetzung nach einem der Ansprüche 1 bis 5, dadurch gekennzeichnet, daß das Olefinsulfonat und das Alkylarysulfonat Natriumsalze sind.

7. Verwendung einer Zusammensetzung nach einem der Ansprüche 1 bis 6, dadurch gekennzeichnet, daß die wäßrige Lösung wenigstens 25 Gew.-% einer Mischung aus (a) und (b) enthält.

8. Verwendung einer Zusammensetzung nach einem der Ansprüche 1 bis 7, dadurch gekennzeichnet, daß die wäßrige Lösung im wesentlichen 30 Gew.-% einer Mischung aus (a) und (b) enthält.

**Revendications**

1. Utilisation d'une composition d'oléfine sulfonate dans un procédé d'extraction améliorée du pétrole caractérisée en ce que la composition comprend une solution aqueuse contenant 15 à 35 % en poids d'un mélange de (a) un alpha-oléfine sulfonate linéaire en $C_{12-22}$ et (b) un alcoyle aryl sulfonate de formule générale

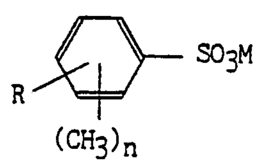

(I)

où n est 0, 1 ou 2, R est un groupe alcoyle en $C_{8-18}$ et M est un ion de métal alcalin ou d'ammonium, le rapport en poids (a):(b) étant compris entre 90:10 et 60:40.

2. Utilisation d'une composition selon la revendication 1 caractérisée en ce que l'oléfine sulfonate est un alpha-oléfine sulfonate linéaire en $C_{14-20}$.

3. Utilisation d'une composition selon la revendication 1 ou 2 caractérisée en ce que l'oléfine sulfonate est un alpha-oléfine linéaire en $C_{16-18}$.

4. Utilisation d'une composition selon la revendication 1, 2 ou 3 caractérisée en ce que dans la formule I, n est 0.

5. Utilisation d'une composition selon l'une quelconque des revendications 1 à 4 caractérisée en ce que R est un groupe alcoyle en $C_{10-14}$.

6. Utilisation d'une composition selon l'une quelconque des revendications 1 à 5 caractérisée en ce que l'oléfine sulfonate et l'alcoyl aryl sulfonate sont des de sodium.

7. Utilisation d'une composition selon l'une quelconque des revendications 1 à 6 caractérisée en ce que la solution aqueuse contient au moins 25 % en poids du mélange de (a) et (b).

8. Utilisation d'une composition selon l'une quelconque des revendications 1 à 7 caractérisée en ce que la solution aqueuse contient environ 30 % en poids du mélange de (a) et (b).